# EUROPEAN PATENT APPLICATION

(11) **EP 4 740 973 A1**
(43) Date of publication of application: **13.05.2026**
(21) Application number: 24861465.3
(22) Date of filing: 20.03.2024
(51) Int. Cl.: A61L 31/04, A61L 31/02

(54) **RELEASE BALLOON CORE FOR DISTAL END RELEASE OF DIGESTIVE TRACT CANNULA, PREPARATION METHOD THEREFOR AND USE THEREOF**

(30) Priority: 07.09.2023 CN 202311152577
(71) Applicant: Hangzhou Tangji Medical Technology Co., Ltd., Hangzhou, Zhejiang 310052 (CN)
(72) Inventor: LI, Wenyu, Hangzhou, Zhejiang 310052 (CN)
(74) Representative: Vogelbruch, Keang
(86) International application number: PCT/CN2024/082732
(87) International publication number: WO 2025/050614

(57) **Abstract**

The present invention provides a release-ball core for distal-end release of a gastrointestinal sleeve, and a preparation method therefor and use thereof, and relates to the technical field of medical devices. The release-ball core includes a filler, a binder, and a disintegrant in a mass ratio of 50-90:10-50:0-20. The filler includes at least one of starch, microcrystalline cellulose, and an inorganic salt. The binder includes at least one of water, ethanol, hydroxypropyl methylcellulose, carboxymethyl cellulose or salts thereof, methylcellulose, and syrup. With gastrointestinal peristalsis and the weight of the release-ball core, the sleeve body is driven to move within the gastrointestinal tract until full self-deployment is achieved. After the self-deployment of the sleeve body, the composition of the release-ball core is controlled to enable detachment from the sleeve body, completing the insertion of the gastrointestinal sleeve. This method provides simple operation, significantly reduces the insertion time of the gastrointestinal sleeve, and lowers the risk of infection and radiation exposure.

## Description

### CROSS-REFERENCE TO RELATED APPLICATIONS

The present invention claims priority to Chinese Patent Application No. 202311152577X, filed with the China National Intellectual Property Administration on September 7, 2023, and entitled "RELEASE-BALL CORE FOR DISTAL-END RELEASE OF GASTROINTESTINAL SLEEVE, AND PREPARATION METHOD THEREFOR AND USE THEREOF", which is incorporated herein by reference in its entirety.

### TECHNICAL FIELD

The present invention relates to the technical field of medical devices, and specifically, to a release-ball core for distal-end release of a gastrointestinal sleeve, and a preparation method therefor and use thereof.

### BACKGROUND

With the deepening and advancing understanding of obesity, it is recognized that obesity is not merely an increase in body weight but accompanied by a symptom of a chronic disease including type 2 diabetes, hypertension, sleep apnea, polycystic ovary syndrome, and the like. Overweight and obesity increase the risk of multiple chronic conditions in adults, which not only lead to severe cardiovascular and cerebrovascular disorders, endocrine and metabolic imbalances, but lead to respiratory, digestive, and musculoskeletal system impairments, and are associated with the development of various malignancies. In addition, obesity also affects mental health and social interactions and contributes to an increased economic burden. The prevalence of obesity-related chronic diseases in China demonstrates a concerning upward trend.

According to WHO standards, with a BMI ≥ 30 kg/m² defining obesity, the prevalence of obesity among Chinese adults increased nearly eightfold between 1980 and 2015. The Report on Nutrition and Health Status of Primary and Secondary School Students in Beijing (2017*)* indicates that in 2017, the prevalence of overweight and obesity among monitored students in Beijing reached 15.9% and 16.9%, respectively, resulting in a combined overweight and obesity prevalence of 32.8%, which aligns with the prevalence observed in the United States. Over the past 20 years, overweight and obesity rates among Chinese residents have increased significantly, surpassing the overweight and obesity rates reported in several developed countries. Current major treatments for obesity include dietary, exercise, and behavioral interventions, pharmacological treatments, and bariatric surgery, which presents a significant challenge due to long duration, and the associated weight loss typically reaches only 3-5%. Although pharmacological treatments can provide additional weight loss benefits, data from electronic medical records in the United States indicate that prescription rates and continuous usage of weight loss drugs remain low due to drug-related adverse effects. Bariatric surgery demonstrates higher efficacy in achieving weight reduction. However, bariatric surgery involves irreversible physiological changes, has a certain risk of mortality, and may result in postoperative complications, such as gastrointestinal leakage, anastomotic stricture, or dumping syndrome. In recent years, inspired by the principles of gastric bypass surgery for weight loss, a sleeve has been placed in the duodenum-jejunum segment to isolate chyme from contact with the intestinal tract, thereby reducing intestinal absorption and achieving weight loss, which has attracted wide research interest from scholars.

Chinese Patent Application No. CN109152570 discloses a method for endoscopic insertion of a sleeve. However, this method requires manual connection between a distal end of the sleeve and a distal cap, and injection of fluid into the sleeve to enable proper extension and shaping of the sleeve within a gastrointestinal tract. The method involves complex operations, prolonged sleeve insertion time, and needs to be operated under X-ray, which significantly increases the risk of infection and radiation hazards. Therefore, there is an urgent need for a novel method for gastrointestinal sleeve release to overcome the above problems.

In view of this, the present invention is proposed.

### SUMMARY

An objective of the present invention is to provide a release-ball core for distal-end release of a gastrointestinal sleeve, and a preparation method therefor and use thereof, where the release-ball core is capable of self-deploying within the gastrointestinal tract, is simple and convenient to operate, and significantly shortens an insertion time of the gastrointestinal sleeve.

Embodiments of the present invention are implemented as follows.

In a first aspect, the present invention provides a release-ball core for distal-end release of a gastrointestinal sleeve. The release-ball core includes a filler, a binder, and a disintegrant in a mass ratio of 50-90:10-50:0-20.

The filler includes at least one of starch, microcrystalline cellulose, and an inorganic salt.

The binder includes at least one of water, ethanol, hydroxypropyl methylcellulose, carboxymethyl cellulose or salts thereof, methylcellulose, and syrup.

In an optional embodiment, the inorganic salt includes at least one of calcium sulfate, dicalcium phosphate, calcium carbonate, and barium sulfate.

Preferably, the barium sulfate is any one of heavy barium sulfate, type I barium sulfate, and type II barium sulfate.

More preferably, the barium sulfate is heavy barium sulfate or type II barium sulfate.

In an optional embodiment, the filler includes either one of the following combinations: microcrystalline cellulose and calcium carbonate, or microcrystalline cellulose and barium sulfate. Preferably, the filler includes microcrystalline cellulose and barium sulfate.

Preferably, a mass ratio of the filler to the binder ranges from 56.2-86.9:13.1-42.2.

In an optional embodiment, the disintegrant includes at least one of crosslinked sodium carboxymethyl starch, crosslinked polyvinylpyrrolidone, crosslinked sodium carboxymethyl cellulose, and sodium chloride.

In an optional embodiment, the release-ball core further includes an excipient, and a mass ratio of the filler to the excipient ranges from 50-90:0.01-5.

Preferably, the excipient includes at least one of an antioxidant, a preservative, and a flavoring agent.

In a second aspect, the present invention provides a preparation method of the release-ball core for distal-end release of a gastrointestinal sleeve according to any one of the foregoing embodiments. The preparation method includes mixing raw materials in a predetermined proportion followed by tableting. The tableting method includes any one of a wet granulation tableting method, a direct compression method, and a molding method.

Preferably, the tableting method is a wet granulation tableting method or a molding method. More preferably, the tableting method is a wet granulation tableting method.

In a third aspect, the present invention provides a gastrointestinal sleeve. The gastrointestinal sleeve includes a sleeve body and the release-ball core for distal-end release of a gastrointestinal sleeve according to any one of the foregoing embodiments. The release-ball core is fixed within a release ball having a lumen. The sleeve body includes a proximal end and a distal end, and the distal end is connected to the release-ball core.

In an optional embodiment, a dry-state connection force between the release-ball core and the distal end of the gastrointestinal sleeve is greater than 2.5 N. Preferably, the dry-state connection force is greater than 5 N.

Preferably, after maintaining a wet state for 2 h, a wet-state connection force between the release-ball core and the distal end of the gastrointestinal sleeve is less than 1.5 N. More preferably, the wet-state connection force is less than 1 N.

In a fourth aspect, the present invention provides use of the release-ball core for distal-end release of a gastrointestinal sleeve according to any one of the foregoing embodiments, or use of the gastrointestinal sleeve according to the foregoing embodiments, in preparation of a product for reducing gastrointestinal absorption.

The embodiments of the present invention have the following beneficial effects:

The present invention provides a release-ball core for distal-end release of a gastrointestinal sleeve, and a preparation method therefor and use thereof. During a release process, natural peristalsis of the intestinal tract drives extension of the sleeve. By controlling the composition of the release-ball core, the release-ball core undergoes disintegration by virtue of the mucus in the distal intestinal tract after full deployment, losing its structural integrity and mechanical properties, thereby causing disengagement of a connection between the sleeve and the release-ball core. The entire release process does not require additional manual operations by medical personnel. The operation method is simple and convenient, significantly shortens a sleeve insertion procedure, and reduces risks associated with infection and X-ray radiation hazards.

### BRIEF DESCRIPTION OF THE DRAWINGS

To more clearly illustrate the technical solutions of the embodiments of the present invention, the drawings required in the embodiments will be briefly described below. It should be understood that the following drawings only illustrate some embodiments of the present invention and therefore should not be considered as limitations of the scope, and for those of ordinary skill in the art, other related drawings can be obtained according to these drawings without creative efforts.
FIG. 1 is a schematic diagram of a structure of a gastrointestinal sleeve according to the present invention;
FIG. 2 is a schematic diagram of a delivery process of the gastrointestinal sleeve according to the present invention; and
FIG. 3 is a digital gastrointestinal radiographic image obtained 4 h after release of the gastrointestinal sleeve according to Test Example 2 of the present invention.

Reference numerals: 100. gastrointestinal sleeve; 110. sleeve body; and 120. release ball.

### DETAILED DESCRIPTION OF THE EMBODIMENTS

The embodiments of the present invention are described in detail below with reference to the examples. Those skilled in the art will understand that the following examples are provided for illustrative purposes only and should not be construed as limiting the scope of the present invention. Unless otherwise specified, the experimental conditions described in the examples are conducted under conventional conditions or in accordance with conditions recommended by manufacturers. Unless otherwise indicated, reagents and instruments used in the examples are commercially available products.

In the present invention, endpoints of disclosed ranges and any individual values are not limited to the exact ranges or values. The disclosed ranges and values should be understood to encompass values close to such ranges or values. For numerical ranges, endpoint values of different ranges, endpoint values and individual point values, and individual point values may be combined with each other to derive one or more new numerical ranges, and such numerical ranges shall be deemed to be specifically disclosed herein.

The present invention provides a release-ball core for distal-end release of a gastrointestinal sleeve. The release-ball core includes a filler, a binder, and a disintegrant in a mass ratio of 50-90:10-50:0-20.

The filler includes at least one of starch, microcrystalline cellulose, and an inorganic salt.

The binder includes at least one of water, ethanol, hydroxypropyl methylcellulose, carboxymethyl cellulose or salts thereof, methylcellulose, and syrup.

Currently, the insertion of gastrointestinal sleeves is mostly performed by injecting a fluid to deploy the sleeve. However, the process of injecting the fluid and waiting for the sleeve to deploy is relatively long, which prolongs the insertion time of the gastrointestinal sleeve and increases the operational difficulty. Accordingly, the inventors have creatively proposed providing a release-ball core on the gastrointestinal sleeve, where the particle can, by virtue of its own weight, drive the sleeve body to self-deploy within the gastrointestinal tract. As a result, the gastrointestinal sleeve can be inserted directly without waiting for a long time for full deployment, thereby making the operation simpler and more convenient. However, the release-ball core must be sterilized prior to use, and sterilization is generally performed using substances such as ethylene oxide. Conventional sustained-release structures or disintegrating particles often exhibit significantly altered disintegration performance after sterilization.

For example, a product that originally disintegrates within approximately 2 h may still fail to disintegrate even 24 h after ethylene oxide sterilization. Therefore, ensuring that the release-ball core maintains favorable disintegration performance after sterilization constitutes a critical factor in achieving the above-described effect of positioning the gastrointestinal sleeve without prolonged waiting for full deployment and enabling a simplified operation.

The inventors have proposed a method of formulating the release-ball core by combining a filler, a binder, and a disintegrant, and by screening raw material compositions and ratios. The resulting release-ball core maintains favorable disintegration performance even after sterilization. The disintegration mechanism of the present invention mainly involves two aspects. The first aspect is capillary-driven disintegration: the release-ball core contains numerous capillaries and pores. When the release-ball core comes into contact with water, the water enters the interior through these hydrophilic channels. Strong water absorption promotes wetting and disintegration of the release-ball core, and the disintegration of cellulose derivatives is often related to this mechanism. The second aspect is dissolution-induced disintegration: when the release-ball core encounters intestinal fluid in the duodenum, soluble components within the release-ball core, such as disintegrants, dissolve in water and form eroding pores. Formation of these eroding pores causes the core to fracture into small particles, and subsequent dissolution of these components accelerates disintegration.

In an optional embodiment, the inorganic salt includes at least one of calcium sulfate, dicalcium phosphate, calcium carbonate, and barium sulfate.

Preferably, the barium sulfate is any one of heavy barium sulfate, type I barium sulfate, and type II barium sulfate. Barium sulfate exhibits radiopacity. Therefore, when the filler of the release-ball core is barium sulfate, the release-ball core provided by the present invention exhibits radiopaque properties. During insertion of the gastrointestinal sleeve, the radiopaque release-ball core allows clear observation of the position of the release-ball core within the gastrointestinal tract, facilitating verification of correct insertion of the gastrointestinal sleeve.

More preferably, the barium sulfate is heavy barium sulfate or type II barium sulfate.

In an optional embodiment, the filler includes either one of the following combinations: microcrystalline cellulose and calcium carbonate, or microcrystalline cellulose and barium sulfate. Preferably, the filler includes microcrystalline cellulose and barium sulfate.

Preferably, a mass ratio of the filler to the binder ranges from 56.2-86.9:13.1-42.2.

In an optional embodiment, to further control the separation time between the release-ball core and the sleeve body, the mass ratio of the filler, the binder, and the disintegrant is 50-90:10-50:0.01-20.

Preferably, the mass ratio of the filler, the binder, and the disintegrant is 56.2-86.9:13.1-42.2:0.01-15.6.

Preferably, the disintegrant includes at least one of crosslinked sodium carboxymethyl starch, crosslinked polyvinylpyrrolidone, crosslinked sodium carboxymethyl cellulose, and sodium chloride.

In an optional embodiment, the mass ratio of the filler, the binder, the disintegrant, and the excipient is 50-90:10-50:0-20:0.01-5.

Preferably, the mass ratio of the filler, the binder, the disintegrant, and the excipient is 56.2-86.9:13.1-42.2:0.01-15.6:0.01-3.

Preferably, the excipient includes at least one of an antioxidant, a preservative, and a flavoring agent.

In a second aspect, the present invention provides a preparation method of the release-ball core for distal-end release of a gastrointestinal sleeve according to any one of the foregoing embodiments. The preparation method includes mixing raw materials in a predetermined proportion followed by tableting. The tableting method includes any one of a wet granulation tableting method, a direct compression method, and a molding method.

Preferably, the tableting method is a wet granulation tableting method or a molding method. More preferably, the tableting method is a wet granulation tableting method. Specific procedures may be performed by referring to conventional tableting techniques.

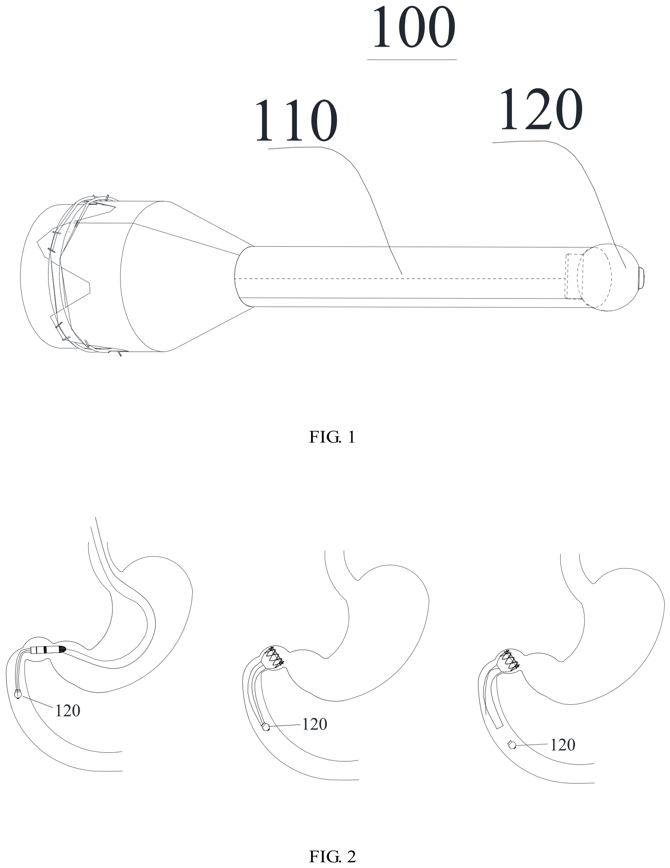

In a third aspect, the present invention provides a gastrointestinal sleeve 100, as shown in FIG. 1. The gastrointestinal sleeve 100 includes a sleeve body 110 and a release-ball core for distal-end release of a gastrointestinal sleeve according to any one of the foregoing embodiments. The release-ball core is fixed within a release ball 120 having a lumen, and the release ball 120 is connected to the sleeve body 110 through the release-ball core. The sleeve body 110 includes a proximal end and a distal end, with the distal end connected to the release-ball core. Further, as shown in FIG. 2, from left to right, the figure illustrates the process of detachment of the release-ball core from the sleeve body 110. First, a delivery tube delivers the gastrointestinal sleeve 100 to the duodenal bulb. The release-ball core, driven by gravity, moves the sleeve body 110 to extend in a direction away from the duodenal bulb. Then, the delivery tube disengages from the sleeve body 110. The release-ball core continues to drive extension of the sleeve body 110 until full deployment is achieved. Finally, the release-ball core disintegrates, and the release ball 120 detaches from the sleeve body 110, completing the gastrointestinal sleeve 100 delivery procedure.

In an optional embodiment, to ensure proper detachment, a dry-state connection force between the release-ball core and the distal end of the gastrointestinal sleeve is greater than 2.5 N. Preferably, the dry-state connection force is greater than 5 N.

Preferably, after maintaining a wet state for 2 h, a wet-state connection force between the release-ball core and the distal end of the gastrointestinal sleeve is less than 1.5 N. More preferably, the wet-state connection force is less than 1 N.

In a fourth aspect, the present invention provides use of the release-ball core for a gastrointestinal sleeve according to any one of the foregoing embodiments, or use of the gastrointestinal sleeve according to the foregoing embodiments, in preparation of a product for reducing gastrointestinal absorption.

### Example 1

This example provides a release-ball core for distal-end release of a gastrointestinal sleeve. The release-ball core includes a filler, a binder, and a disintegrant.

The filler is 2 g of microcrystalline cellulose and 2 g of type II barium sulfate. The binder is 2 g of water and 0.12 g of carboxymethyl cellulose. The disintegrant is 0.1 g of crosslinked sodium carboxymethyl cellulose.

The above raw materials were mixed according to the specified proportions and subjected to wet granulation tableting, with a tableting pressure of 0.4 MPa. The release-ball core was prepared after ethylene oxide sterilization.

This example also provides a gastrointestinal sleeve, including a sleeve body, and the sleeve body includes a proximal end and a distal end. The release-ball core, after ethylene oxide sterilization, is fixedly connected to the distal end of the sleeve body.

### Example 2

This example provides a release-ball core for distal-end release of a gastrointestinal sleeve. The release-ball core includes a filler, a binder, and a disintegrant.

The filler is 2 g of microcrystalline cellulose and 2 g of heavy barium sulfate. The binder is 1.2 g of water and 1 g of carboxymethyl cellulose. The disintegrant is 0.1 g of crosslinked sodium carboxymethyl cellulose. The above raw materials were mixed according to the specified proportions and subjected to wet granulation tableting in the same manner as described in Example 1. The release-ball core was prepared after ethylene oxide sterilization.

This example also provides a gastrointestinal sleeve, including a sleeve body, and the sleeve body includes a proximal end and a distal end. The release-ball core after sterilization, is fixedly connected to the distal end of the sleeve body.

### Example 3

This example provides a release-ball core for distal-end release of a gastrointestinal sleeve. The release-ball core includes a filler, a binder, and a disintegrant.

The filler is 1 g of microcrystalline cellulose and 2 g of type II barium sulfate. The binder is 1.2 g of water and 2 g of carboxymethyl cellulose. The disintegrant is 0.3 g of crosslinked sodium carboxymethyl cellulose. The above raw materials were mixed according to the specified proportions and subjected to wet granulation tableting in the same manner as described in Example 1. The release-ball core was prepared after ethylene oxide sterilization.

This example also provides a gastrointestinal sleeve, including a sleeve body, and the sleeve body includes a proximal end and a distal end. The release-ball core after sterilization, is fixedly connected to the distal end of the sleeve body.

### Example 4

This example provides a release-ball core for distal-end release of a gastrointestinal sleeve. The release-ball core includes a filler, a binder, and a disintegrant.

The filler is 2 g of microcrystalline cellulose and 2 g of type II barium sulfate. The binder is 1.2 g of water and 0.2 g of carboxymethyl cellulose. The disintegrant is 0.3 g of crosslinked sodium carboxymethyl cellulose. The above raw materials were mixed according to the specified proportions and subjected to wet granulation tableting in the same manner as described in Example 1. The release-ball core was prepared after ethylene oxide sterilization.

This example also provides a gastrointestinal sleeve, including a sleeve body, and the sleeve body includes a proximal end and a distal end. The release-ball core after sterilization, is fixedly connected to the distal end of the sleeve body.

### Example 5

This example provides a release-ball core for distal-end release of a gastrointestinal sleeve. The release-ball core includes a filler, a binder, and a disintegrant.

The filler is 0.5 g of microcrystalline cellulose and 3.5 g of type II barium sulfate. The binder is 1 g of water and 0.12 g of carboxymethyl cellulose. The disintegrant is 0.1 g of sodium chloride. The above raw materials were mixed according to the specified proportions and subjected to wet granulation tableting in the same manner as described in Example 1. The release-ball core was prepared after ethylene oxide sterilization.

This example also provides a gastrointestinal sleeve, including a sleeve body, and the sleeve body includes a proximal end and a distal end. The release-ball core after sterilization, is fixedly connected to the distal end of the sleeve body.

### Example 6

This example provides a release-ball core for distal-end release of a gastrointestinal sleeve. The release-ball core includes a filler, a binder, and a disintegrant.

The filler is 1 g of microcrystalline cellulose and 3 g of heavy barium sulfate. The binder is 1 g of water and 0.12 g of carboxymethyl cellulose. The disintegrant is 0.1 g of sodium chloride. The above raw materials were mixed according to the specified proportions and subjected to molding tableting. The release-ball core was prepared after ethylene oxide sterilization.

This example also provides a gastrointestinal sleeve, including a sleeve body, and the sleeve body includes a proximal end and a distal end. The release-ball core after sterilization, is fixedly connected to the distal end of the sleeve body.

### Example 7

This example provides a release-ball core for distal-end release of a gastrointestinal sleeve. The release-ball core includes a filler, a binder, and a disintegrant.

The filler is 2.5 g of microcrystalline cellulose and 2 g of type II barium sulfate. The binder is 1.2 g of water and 0.5 g of carboxymethyl cellulose. The disintegrant is 0.1 g of sodium chloride. The above raw materials were mixed according to the specified proportions and subjected to direct tableting. The release-ball core was prepared after ethylene oxide sterilization.

This example also provides a gastrointestinal sleeve, including a sleeve body, and the sleeve body includes a proximal end and a distal end. The release-ball core after sterilization, is fixedly connected to the distal end of the sleeve body.

### Example 8

This example provides a release-ball core for distal-end release of a gastrointestinal sleeve. The release-ball core includes a filler, a binder, and a disintegrant.

The filler is 2 g of microcrystalline cellulose and 2.8 g of type II barium sulfate. The binder is 1 g of water, 0.2 g of ethanol, and 0.12 g of carboxymethyl cellulose. The disintegrant is 0.3 g of crosslinked sodium carboxymethyl cellulose. The above raw materials were mixed according to the specified proportions and subjected to direct tableting. Then, 0.2 g of sodium chloride was added during tableting. The release-ball core was prepared after ethylene oxide sterilization.

This example also provides a gastrointestinal sleeve, including a sleeve body, and the sleeve body includes a proximal end and a distal end. The release-ball core after sterilization, is fixedly connected to the distal end of the sleeve body.

### Comparative Example 1

This comparative example provides a gastrointestinal sleeve, which is identical to the gastrointestinal sleeve of Example 1, except that the release-ball core was prepared using raw materials without a disintegrant.

### Comparative Example 2

This comparative example provides a gastrointestinal sleeve, which is identical to the gastrointestinal sleeve of Example 2, except that the release-ball core was prepared using raw materials without a disintegrant.

### Comparative Example 3

This comparative example provides a gastrointestinal sleeve, which is identical to the gastrointestinal sleeve of Example 3, except that the release-ball core was prepared using raw materials without a disintegrant.

### Comparative Example 4

This comparative example provides a gastrointestinal sleeve, which is identical to the gastrointestinal sleeve of Example 4, except that the release-ball core was prepared using raw materials without a disintegrant.

### Comparative Example 5

This comparative example provides a gastrointestinal sleeve, which is identical to the gastrointestinal sleeve of Example 5, except that the release-ball core was prepared using raw materials without a disintegrant.

### Comparative Example 6

This comparative example provides a gastrointestinal sleeve, which is identical to the gastrointestinal sleeve of Example 6, except that the release-ball core was prepared using raw materials without a disintegrant.

### Comparative Example 7

This comparative example provides a gastrointestinal sleeve, which is identical to the gastrointestinal sleeve of Example 7, except that the release-ball core was prepared using raw materials without a disintegrant.

### Comparative Example 8

This comparative example provides a gastrointestinal sleeve, which is identical to the gastrointestinal sleeve of Example 8, except that the release-ball core was prepared using raw materials without a disintegrant.

### Comparative Example 9

This comparative example provides a release-ball core for a gastrointestinal sleeve. The difference from Example 1 is that the disintegrant is dry starch.

### Comparative Example 10

This comparative example provides a release-ball core for a gastrointestinal sleeve. The difference from Example 2 is that the disintegrant is crosslinked polyvinylpyrrolidone.

### Comparative Example 11

This comparative example provides a release-ball core for a gastrointestinal sleeve. The difference from Example 3 is that the disintegrant is low-substituted hydroxypropyl cellulose.

### Test Example 1

The gastrointestinal sleeves provided in Examples 1-8 and Comparative Examples 1-11 were tested for disintegration time in physiological saline, dry-state connection force, and wet-state connection force.

The testing methods were as follows:
1. Disintegration time: Determined according to the method described in the 2020 edition of the Pharmacopoeia of the People's Republic of China, Section 0921, Disintegration Test.
2. Dry-state connection force: The release ball and the membrane tube were clamped separately in a universal testing machine. The release ball was pulled at a speed of 20 mm/min until detachment from the membrane tube. The maximum force at detachment was recorded.
3. Wet-state connection force: The release ball was immersed in physiological saline and oscillated in a water bath at 37±2 °C with a shaking speed of 40 rpm for 2 h. After treatment, the release ball and the membrane tube were clamped separately in a universal testing machine. The release ball was pulled at a speed of 20 mm/min until detachment from the membrane tube. The maximum force at detachment was recorded.

The results are shown in Table 1.

**Table 1 Detachment time of release balls from sleeve bodies of different shapes**

| | Disintegration time | Dry-state connection force (N) | Wet-state connection force (N) |
|---|---|---|---|
| Example 1 | <15 min | 8.321 | 0.714 |
| Example 2 | <15 min | 7.823 | 0.543 |
| Example 3 | <15 min | 9.651 | 0.324 |
| Example 4 | <15 min | 10.318 | 0.381 |
| Example 5 | <15 min | 13.189 | 0.218 |
| Example 6 | <15 min | 13.892 | 0.461 |
| Example 7 | <15 min | 7.325 | 0.192 |
| Example 8 | <15 min | 9.32 | 0.362 |
| Comparative Example 1 | >2 h | 8.062 | 7.875 |
| Comparative Example 2 | >2 h | 7.54 | 7.201 |
| Comparative Example 3 | 100 min | 9.121 | 8.356 |
| Comparative Example 4 | >2 h | 10.288 | 9.176 |
| Comparative Example 5 | >2 h | 13.201 | 11.912 |
| Comparative Example 6 | >2 h | 13.223 | 12.856 |
| Comparative Example 7 | >2 h | 6.711 | 5.988 |
| Comparative Example 8 | >2 h | 8.806 | 7.892 |
| Comparative Example 9 | >2 h | 10.212 | 5.828 |
| Comparative Example 10 | >1 h | 9.804 | 4.852 |
| Comparative Example 11 | >2 h | 12.321 | 6.282 |

As shown in Table 1, the release-ball cores of the embodiments of the present invention exhibit shorter disintegration times and better disintegration performance. This characteristic facilitates complete self-deployment of the sleeve body and allows the gastrointestinal sleeve to be used without prolonged blockage of the sleeve body.

### Test Example 2

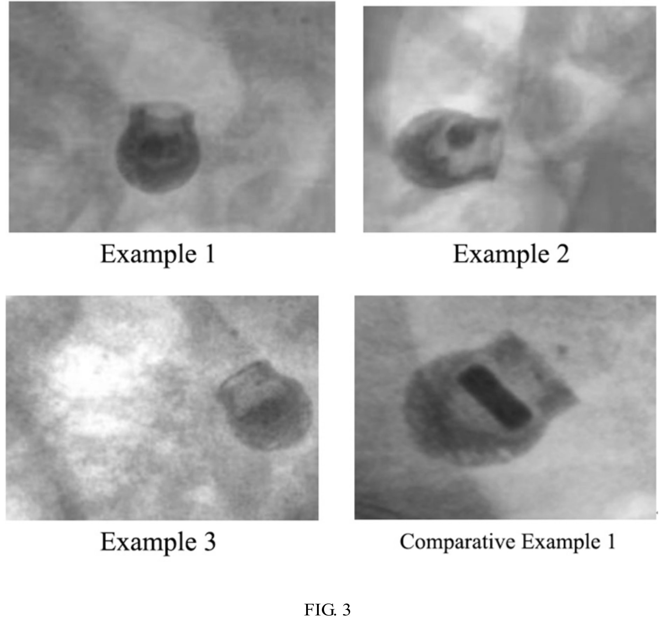

The gastrointestinal sleeves prepared in Examples 1-3 and Comparative Example 1 were inserted into Bama miniature pigs. The disintegration time of the release-ball cores prepared in Examples 1-3 and Comparative Example 1 in vivo was evaluated. X-ray imaging was used to observe the detachment time of the release balls from the membrane tubes in the Bama miniature pigs. The results are shown in FIG. 3. As shown in FIG. 3, the release-ball cores of the embodiments of the present invention were able to detach from the sleeve bodies in vivo by controlling the combination and proportion of the raw materials. In contrast, the release-ball core of Comparative Example 1 did not disintegrate and could not detach from the sleeve body. As a result, the X-ray image showed a dark shadow representing the connection between the release-ball core and the sleeve body, which is unfavorable for the use of the gastrointestinal sleeve.

The above described contents are only optional embodiments of the present invention and are not intended to limit the present invention. For those skilled in the art, the present invention can be modified and varied. Any modification, equivalent replacement, improvement, or the like made without departing from the spirit and principle of the present invention shall fall within the protection scope of the present invention.

### Industrial Applicability

The present invention is capable of self-deployment within the gastrointestinal tract, provides simple and convenient operation, significantly reduces the insertion time of the gastrointestinal sleeve, and possesses high practical value.

## Claims

1. A release-ball core for distal-end release of a gastrointestinal sleeve, **characterized by** comprising a filler, a binder, and a disintegrant in a mass ratio of 50-90:10-50:0-20, wherein
the filler comprises at least one of starch, microcrystalline cellulose, and an inorganic salt; and
the binder comprises at least one of water, ethanol, hydroxypropyl methylcellulose, carboxymethyl cellulose or salts thereof, methylcellulose, and syrup.

2. The release-ball core for distal-end release of a gastrointestinal sleeve according to claim 1, wherein the inorganic salt comprises at least one of calcium sulfate, dicalcium phosphate, calcium carbonate, and barium sulfate;
preferably, the barium sulfate is any one of heavy barium sulfate, type I barium sulfate, and type II barium sulfate; and
more preferably, the barium sulfate is heavy barium sulfate or type II barium sulfate.

3. The release-ball core for distal-end release of a gastrointestinal sleeve according to claim 2, wherein the filler comprises either one of the following combinations: microcrystalline cellulose and calcium carbonate, or microcrystalline cellulose and barium sulfate; preferably, the filler comprises microcrystalline cellulose and barium sulfate; and
preferably, a mass ratio of the filler to the binder is 56.2-86.9:13.1-42.2.

4. The release-ball core for distal-end release of a gastrointestinal sleeve according to claim 1, wherein the disintegrant comprises at least one of crosslinked sodium carboxymethyl starch, crosslinked polyvinylpyrrolidone, crosslinked sodium carboxymethyl cellulose, and sodium chloride.

5. The release-ball core for distal-end release of a gastrointestinal sleeve according to claim 1 or 4, further comprising an excipient, wherein a mass ratio of the filler to the excipient is 50-90:0.01-5.

6. The release-ball core for distal-end release of a gastrointestinal sleeve according to claim 5, wherein the excipient comprises at least one of an antioxidant, a preservative, and a flavoring agent.

7. A preparation method of the release-ball core for distal-end release of a gastrointestinal sleeve according to any one of claims 1 to 6, **characterized by** comprising mixing raw materials in a predetermined proportion followed by tableting, wherein the tableting method comprises any one of a wet granulation tableting method, a direct compression method, and a molding method;
preferably, the tableting method is a wet granulation tableting method or a molding method; and more preferably, the tableting method is a wet granulation tableting method.

8. A gastrointestinal sleeve, **characterized by** comprising a sleeve body and the release-ball core for distal-end release of a gastrointestinal sleeve according to any one of claims 1 to 6, wherein the release-ball core is fixed within a release ball having a lumen, the sleeve body comprises a proximal end and a distal end, and the distal end is connected to the release-ball core.

9. The gastrointestinal sleeve according to claim 8, wherein a dry-state connection force between the release-ball core and the distal end of the gastrointestinal sleeve is greater than 2.5 N, preferably, the dry-state connection force is greater than 5 N;
preferably, after maintaining a wet state for 2 h, a wet-state connection force between the release-ball core and the distal end of the gastrointestinal sleeve is less than 1.5 N; and more preferably, the wet-state connection force is less than 1 N.

10. Use of the release-ball core for distal-end release of a gastrointestinal sleeve according to any one of claims 1-6, or the gastrointestinal sleeve according to claim 8 or 9, after ethylene oxide sterilization, in a product for reducing gastrointestinal absorption.
